# EUROPEAN PATENT APPLICATION

(11) **EP 3 685 669 A1**
(43) Date of publication of application: **29.07.2020**
(21) Application number: 19153405.6
(22) Date of filing: 23.01.2019
(51) Int. Cl.: A01N 63/02, A61K 35/742, A61P 31/00, A61P 31/04, A61P 17/02, A01P 1/00

(54) **COMPOSITION FOR THE DECONTAMINATION AND INOCULATION OF SURFACES**

(71) Applicant: Chrisal NV, 3920 Lommel (BE)
(72) Inventor: GIELEN, Corrie, B-3900 Overpelt (BE); TEMMERMAN, Robin, B-9290 Berlare (BE)
(74) Representative: IPLodge bv

(57) **Abstract**

The invention relates to a composition for the decontamination and inoculation of surfaces, said composition comprising an antimicrobial agent and a mixture of stabilized *Bacteria.* Said composition can be used for inoculating said surfaces for the purpose of purifying said surfaces of unwanted, harmful or pathogenic micro-organisms.

## Description

### Field of the invention

The present invention relates to compositions for the decontamination and inoculation of surfaces. The invention also relates to a corresponding method for use in same.

### Introduction

It is known that in some cases, traditional cleaning products and methods are not sufficient or efficient enough. In e.g. hospitals, equipment such as furniture, floors, and patient beds and mattresses require regular and thorough cleaning to reduce the number of pathogenic microorganisms which may affect the health of recovering patients. Furthermore, for obvious reasons, shared facilities, such as industrial kitchens and components thereof as well as industrial facilities and components, such as filters, pumps and ducts, also need a permanent control and reduction of such pathogenic populations.

It is known that probiotics can be used in combating these harmful bacteria. WO 2006/125283 teaches that the colonizing of surfaces with non-pathogenic spore-forming bacteria via cleaning products has a favourable effect in reducing the number of pathogenic microorganisms on such surfaces. This decreases the risk of infections and has a beneficial effect on general health.

However, in view of the growing resistance of some bacterial strains against certain cleaning products, it is extremely important to reduce the numbers of such pathogens as much as possible. There is therefore a need for products and methods that can reduce any unwanted, harmful or pathogenic microorganisms on a surface to a minimum.

### Summary of the invention

According to an aspect of the present invention, there is provided a composition for the decontamination and inoculation of surfaces, said composition comprising an antimicrobial agent and probiotic component, said probiotic component being a mixture of stabilized *Bacteria.*

It is an advantage of the present invention that the composition according to the invention can be used to reduce populations of harmful or pathogenic microorganisms. Furthermore, it has been found that the combination of an antimicrobial agent, such as a disinfectant, and a probiotic component has an invigorative effect on the reduction of these pathogenic population concentrations with respect to decontamination processes on the basis of a product having an antimicrobial agent or a probiotic component only. It was furthermore advantageously found that repeated cycles of decontamination using said composition result, in an accelerated manner, in a permanently low pathogen population concentration on the surface, as compared to surface decontamination with compositions having an antimicrobial agent or a probiotic component only.

In an embodiment of the composition according to the present invention, said probiotic component comprises at least one of *Bacillus* spores, Lactic Acid *Bacteria*, soil *Bacteria*, water *Bacteria*, human microbiome *Bacteria* or plant *Bacteria.*

In an embodiment of the composition according to the present invention, said probiotic component is a mixture of *Bacillus* spores.

It is an advantage of this embodiment that spores of species of the genus *Bacillus* are easy to manipulate and can be used in a product having a shelf life of at least three years.

In an embodiment of the composition according to the present invention, said mixture of *Bacillus* spores is present in a concentration of 1 x 10⁶ to 1 x 10¹⁰ cfu per gram composition.

It is an advantage of this embodiment that said concentration range allows both decontaminating surfaces by use of a detergent or cleaning product comprising said composition, as well as decontaminating surfaces by water dosing, wherein products for the latter typically have an initially higher cfu concentration per gram composition due to the fact that these products tend to be diluted upon use.

In an embodiment of the composition according to the present invention, said composition further comprises at least one prebiotic agent.

It is an advantage of this embodiment that the use of at least one prebiotic agent results in a further reduction of the pathogen population concentration. Furthermore, adding at least one prebiotic agent to a probiotic component has been found to increase the speed of germination - and thus of activity - of the latter.

In an embodiment of the composition according to the present invention, said at least one prebiotic agent comprises at least one of a fructo-oligosaccharide, a galacto-oligosaccharide and/or inulin.

In an embodiment of the composition according to the present invention, said at least one prebiotic agent is present in a concentration of 100 mg to 100 g per kg composition.

In an embodiment of the composition according to the present invention, the antimicrobial agent is selected from the group consisting of alcohols, aldehydes, oxidizing agents, peroxy acids, phenolics, quaternary ammonium compounds, chlorine compounds, iodine compounds, acid and bases, metals and terpenes, or mixtures thereof.

In an embodiment of the composition according to the present invention, said antimicrobial agent is present in a concentration of 0,001wt% to 90 wt% with reference to the total weight of the composition.

In an embodiment of the composition according to the present invention, said probiotic component and said prebiotic agent are at least partly encapsulated in microcapsules.

It is an advantage of this embodiment that the use of microcapsules further allows to spread germination in time, thereby ensuring a constant presence and a persistent activity of *Bacteria* on the treated surface.

In an embodiment of the composition according to the present invention, said microcapsule comprises a brittle shell and is furthermore filled with a non-aqueous solution.

In an embodiment of the composition according to the present invention, said composition is formulated as a detergent, cleaning product, liquid, emulsion, cream, ointment, lotion, gel, oil, solution, aerosol spray, powder, or semi-solid formulation.

It is an advantage of this embodiment that said composition can be used as part of or formulated as the above-mentioned products, thereby increasing the field of application of the composition. Advantageously, said products typically have a shelf life of at least three years.

In an embodiment of the composition according to the present invention, the surface is wood, paper or textile; the surface is an abiotic material, such as stone, metal, plastic, glass, carbon, or ceramic; or the surface is a composite material.

It is an advantage of this embodiment that said composition can be applied on a variety of surfaces, which are typically abiotic. However, with the exception of human or animal skin, said composition can also be applied on some biotic materials.

According to an aspect of the present invention, there is provided a method for treating surfaces with the composition for the simultaneous decontamination and inoculation of surfaces, said method comprising the steps of cleaning, spraying, ultrasound fogging, painting, or water dosing said surfaces with said composition.

According to an aspect of the present invention, there is provided a cleaning product comprising said composition.

### Brief description of the figures

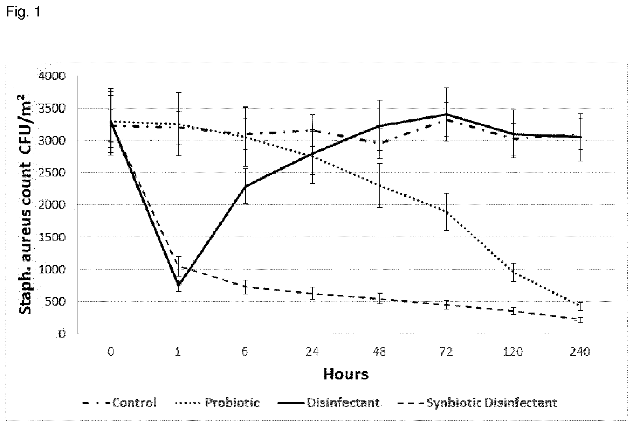

Figure 1 shows the effect of cleaning products on the count of Staphylococcus aureus populations.

### Detailed Description of Embodiments

The invention described herein refers to a composition for the decontamination and inoculation of surfaces, said composition comprising an antimicrobial agent and a probiotic component.

A specific application of the composition according to embodiments of the invention is a composition for the decontamination and inoculation of surfaces of non-living things. It is thereby noted that the term "non-living thing" can also refer to that which used to be part of a living thing, such as wood, rubber, paper and the like. However, the term "surface" does not refer herein to the surface of living beings, thereby excluding human skin, animal skin or fur and the like.
More in particular, such a surface can be a surface made of or including wood, paper, leather, textile; the surface can be a surface made of or including an abiotic material, such as stone, metal (and alloys thereof), plastic and other polymeric materials, glass, ceramic materials, bricks and the like. Furthermore, said surface can be a surface of composite material, the term "composite material" referring herein to material made from two or more constituent materials. Said surface can further be a mixture of the aforementioned materials.
According to embodiments, said surface can relate to a surface which is exposed to air, or which is partially or completely submerged in water or in another liquid.

For the purpose of the invention, the term "decontamination of surfaces" refers herein to acting on a surface with the purpose of purification of that surface, meaning the reduction of unwanted, harmful or pathogenic microorganisms on the surface, such as bacteria, fungi, yeasts, viruses and the like.
For the purpose of the invention, the term "inoculation of surfaces" refers herein to the act of introducing microorganisms, more in particular a probiotic component, onto said surface.
For these purposes, the decontamination and inoculation of surfaces may comprise the act of cleaning the surface with said composition, or by applying said composition to said surface through the act of spraying, ultrasound fogging, painting or water dosing.
For the purpose of the invention, the term "water dosing" refers herein to the purification of surfaces in installations and/or surfaces of components thereof by addition of said composition to a liquid, such as a cooling liquid, a rinsing liquid or a circulating liquid, wherein said liquid is in contact with the surface to be treated. Surfaces of pools and ponds, as well as surfaces of industrial installations, such as the inside surface of ducts, pumps and filters may be subject to biofilm growth and accumulation of organic waste. Said composition may thus be added to the liquid which is in contact with said surface.

Where appropriate, the invention will be described with reference to such applications, without the intent to limit the scope of the invention thereto.

For the purpose of the present invention, the term "antimicrobial agent" refers to an agent that destroys or inhibits the growth of microorganisms, in particular pathogenic microorganisms. Disinfectants are considered a class of antimicrobial agents.

According to embodiments of the invention, said antimicrobial agent is selected from the group consisting of alcohols, aldehydes, oxidizing agents, peroxy acids, phenolics, quaternary ammonium compounds, chlorine compounds, iodine compounds, acid and bases, metals and terpenes, or mixtures thereof.

According to embodiments of the invention, said antimicrobial agent is present in a concentration of at least 0,001 wt%, preferably at least 0.01 wt%, more preferably at least 0.1 wt%, even more preferably at least 1 wt% and most preferably at least 5 wt% with reference to the total weight of the composition. It will further be understood that said antimicrobial agent is present in a concentration of at most 90 wt%, preferably at most 80 wt%, more preferably at most 70 wt%, even more preferably at most 60 wt%, more preferably at most 50 wt%, even more preferably at most 40 wt%, and most preferably at most 30 wt% with reference to the total weight of the composition.

For the purpose of the present invention, the term "synbiotic" refers to a combination of a probiotic and a prebiotic.

For the purpose of the present invention, the terms "prebiotic" or "at least one prebiotic agent" refer to at least one of a short-chain sugar (typically 3-9 monosaccharide units).
According to particular embodiments, the prebiotic contains one or more from the group consisting of galacto-oligosaccharides, fructo-oligosaccharides and inulin.
According to preferred embodiments, the prebiotic is inulin.

According to preferred embodiments of the invention, the at least one prebiotic agent has a mass fraction which is at least 0.5 mg, preferably at least 1 mg, more preferably at least 10 mg, even more preferably at least 100 mg, and most preferably at least 1g, per kg of the composition. It will further be understood that the at least one prebiotic agent has a mass fraction which is at most 300 g, preferably at most 200 g, more preferably at most 100 g and most preferably at most 80 g, per kg of the composition.

For the purpose of the present invention, the term "probiotic" or "probiotic component" refers herein to a mixture of stabilized species of the domain *Bacteria.*

According to embodiments of the invention, said probiotic component comprises at least one of the following: spores of at least one non-pathogenic species of the genus *Bacillus*, Lactic Acid *Bacteria*, soil *Bacteria*, water *Bacteria*, human microbiome *Bacteria* or plant *Bacteria*; such as but not limited to *Lactobacillus, Lactococcus, Bifidobacterium, Azospirillum, Enterobacter*, *Klebsiella*, *Pseudomonas*, *Sphingomonas*, *Nitrospira*, *Pedomicrobium*, *Mycobacterium*, *Nocardia*, *Desulfovibrio* and *Sulfuricurvum.*

According to preferred embodiments of the invention, the probiotic component comprises a mixture of spores of different non-pathogenic *Bacillus* species (referred to as *Bacillus spp.* herein after).

According to preferred embodiments of the invention, said probiotic component comprises at least one of *Bacillus subtilis*, *Bacillus licheniformis*, *Bacillus megaterium* and *Bacillus amyloliquefaciens.*

Preferably, the probiotic component comprises a mixture of spores of *Bacillus subtilis*, *Bacillus licheniformis*, *Bacillus megaterium* and *Bacillus amyloliquefaciens.*

Preferably, for a mixture comprising spores of *Bacillus subtilis*, *Bacillus licheniformis*, *Bacillus megaterium* and *Bacillus amyloliquefaciens,* each of said four *Bacillus* species has a number of spores which is at least 10%, preferably at least 15%, and more preferably at least 20%, with reference to the total number of spores of said four *Bacillus spp.* in the mixture. It is further understood that each of said four *Bacillus* species has a number of spores which is at most 70%, preferably at most 60%, more preferably at most 60%, even more preferably at most 50%, preferably at most 40%, more preferably at most 35%, and most preferably at most 30%, with reference to the total number of spores of said four *Bacillus spp.* in the mixture. Preferably, spores of said four species are present in substantially or exactly equal amounts in the mixture.

According to particular embodiments of the invention, said mixture further comprises *Bacillus polymyxa.*

According to particular embodiments of the invention, said mixture does not comprise *Bacillus coagulans.*

According to preferred embodiments, the total amount of spores in said probiotic component comprising a mixture of spores of different non-pathogenic *Bacillus* species ranges from 1x10⁶ to 1x10¹⁰ per gram of the composition. Such an amount is expressed as cfu (colony-forming units). It is thereby understood that this value refers to the total number of cfu of all the *Bacillus spp.* together.

According to particular embodiments, the probiotic component is a mixture consisting of spores of *Bacillus subtilis*, *Bacillus licheniformis*, *Bacillus megaterium* and *Bacillus amyloliquefaciens.*

The transformation of spores to vegetative cells is the result of absorption of moisture from the surrounding surface or from the biofilm to which the preparation is applied [Knudsen et al. (2015) J. Bacteriol. 19;198(1): 168-77]. This moisture absorption decreases the absolute moisture content of the surface or biofilm, thus exerting an inhibitory action on moisture availability to other microorganisms. The vegetative Bacillus cells produce a variety of extracellular enzymes on surfaces and in biofilm that reduce the amount of organic nutrients on the surface and in biofilm [Priest (1977) Bacteriol. Rev. 41(3): 711-53]. This reduction exerts an inhibitory action on the food availability to other microorganisms via nutrient depletion and the general concept of competitive exclusion [Ragione et al. (2003) Vet. Microbiol. 94(3): 245-56].

Treating a surface with a decontamination composition containing a probiotic component can affect the concentration of unwanted and potentially harmful microorganisms.
In a particularly interesting embodiment, such an effect can be further optimized by a selection of a mixture the aforementioned four *Bacillus spp*., i.e. *Bacillus subtilis*, *Bacillus licheniformis*, *Bacillus megaterium* and *Bacillus amyloliquefaciens.* As these *Bacillus spp.* have a different rate in germination, therefore also a different concentration distribution over time, a constant presence of the probiotic component on the surface to be treated is assured. Said selection will result in an even more pronounced moisture depletion as well as an optimized diversity of extracellular enzymes.

It is an advantage of the present invention that the composition described herein can be used to reduce populations of harmful or pathogenic microorganisms.
More in particular, the combination of an antimicrobial agent, such as a disinfectant, and a probiotic component has as an effect that these pathogenic population concentrations can be reduced to a low level, in comparison with compositions containing a microbial agent or a probiotic component only. Repeating decontamination of surfaces at fixed times with a composition according to the invention, advantageously resulted in a permanent pathogen population concentration, said concentration being low as compared to surface decontamination with compositions having a disinfectant or a probiotic component only.
It was furthermore unexpectedly found that the addition of a prebiotic component to such a composition invigorated the effect of the composition even further, by accelerating the decontaminating effect as well as reducing even further the concentration of pathogens on the treated surface.

The composition for decontamination and inoculation of surfaces can be used to purify a surface from organic dirt, which may be in the form of a biofilm matrix, as well as to lower the number of other microorganisms such bacteria, viruses, yeasts and fungi. It has been found that the addition of at least one prebiotic agent to the probiotic component further strengthens the purifying effect of the composition, the prebiotic component thereby seemingly supporting and strengthening the effect of the probiotics on biofilm removal and lowering other micro-organisms.

According to embodiments of the invention, at least one of the probiotic component, the antimicrobial agent and said at least one prebiotic agent can be at least partly encapsulated in microcapsules.

For the purpose of the invention, microcapsules are particles containing at least one of the probiotic component, the antimicrobial agent and said at least one prebiotic agent.
According to preferred embodiments, said microcapsule comprise the probiotic component and said at least one prebiotic agent. Such capsules are described in US 20120076864. Advantageously, these components can be encapsulated in such microcapsules to provide the composition with longer stability/shelf life. Furthermore, after a "free" first part of the probiotic component and, if present, the at least one prebiotic agent of the composition, which part was not encapsulated in microcapsules, has already exerted its effect, said microcapsules may provide for a delayed release of a second part of the probiotic component and the at least one prebiotic agent on the surface or in biofilm. As a result thereof, a persistent activity of the composition for up to 6 weeks can be obtained, provided that sufficient friction is present.
If the preparation is thereby applied to a surface that suffers from friction (such as human or animal activity (walking, touching) or mechanical activity (interaction with machines or vehicles and the like)), the presence of the microcapsules is an established way of ensuring that the preparation exerts a lasting action.

Consequently, it is advantageous to include such microcapsules in the composition for the decontamination and inoculation of surfaces where frequent treatment of the surface is not possible.

Typically, the microcapsules are composed of a brittle shell comprising a liquid containing the spores. Said shell breaks open due to friction and releases the contents of the capsule. The microcapsules are typically 1 to 300 µm in diameter.

According to embodiments of the invention, the shell is composed of a polymer layer, such as a polymer of gelatin, polyurethane, polyolefins, polyamides, polyesters, polysaccharides, silicone resins, epoxy resins, chitosan and aminoplastic resins such as a melamine formaldehyde resin.

According to embodiments of the invention, said probiotic component and said at least one prebiotic agent may be present together or separately in a microcapsule. However, said microcapsules may also be provided containing individual bacterial strains.

According to embodiments of the invention, the liquid in the capsules is typically non-aqueous, and more specifically is immiscible with water (e.g. an organic oil, a silicone oil, a fluorocarbon, or mixtures thereof). According to particular embodiments, said liquid may contain said antimicrobial agent.

The weight ratio of the shell to the liquid contained therein is typically 1:500 to 1:5000. The outer layer of this shell can contain functional reactive groups for chemical binding.

According to embodiments of the invention, up to 10 wt%, 20 wt%, 30 wt%, 50 wt%, or even 75 wt% of the probiotic component and/or the at least one prebiotic agent may be encapsulated in the microcapsules. Microcapsules may be dispersed in a liquid or viscous composition.

According to embodiments of the invention, the composition is processed into cleaning products (detergents), or products for spraying, for (ultrasound) fogging, for painting or for water dosing. Such products are typically in liquid form.
Furthermore, depending on the type of treatment and surface type in question, the composition may be used as a component in or formulated as: a detergent, a cleaning product, a liquid, an emulsion, a cream, an ointment, a lotion, a gel, an oil, a solution, a trigger spray, an aerosol spray, a powder, or a semi-solid formulation.

Advantageously, said composition can be processed in a product for fogging, preferably ultrasound fogging, and combined with a fogging device. An ultrasound fogging device refers herein to a device that uses high-frequency sound vibrations to produce an extra fine water mist that is then expelled to add moisture to the room. It has been found that such an application allows surface treatment of surfaces that cannot be reached by conventional cleaning techniques or that cannot be treated by means of water dosing.
Compositions for decontaminating and inoculating surfaces by (ultrasound) fogging and methods therefore, may be particularly interesting for the treatment of HVAC installation surfaces, especially the surface of the inner sides of ducts. It was found that the installation of an ultrasound fogging device in the duct of an air-conditioning installation, said device fogging a surface decontaminating composition according to the invention had a beneficial influence on the presence of harmful microorganisms.
In preferred embodiments, water droplets have an average diameter ranging from 0.5 to 10 µm. It was found that under these conditions, the composition could be maximally distributed into an available space.

The invention further relates to a method for treating surfaces with the composition for the simultaneous decontamination and inoculation of surfaces as described here above. Part of said composition may be encapsulated in microcapsules. Said method further comprises the steps of cleaning (in the sense of freeing from dirt of pollution), spraying, ultrasound fogging, painting, or water dosing said surfaces with said composition according to practices known in the state of the art.

The invention further relates to a cleaning product comprising the composition as described herein.

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

### Experimental Results

The invention will be now described in more details with reference to the following examples, whose purpose is merely illustrative and not intended to limit the scope of the invention.
Galacto-oligosaccharides: Ergomax GOS
Fructo-oligosaccharides: Supersmart Fructo
Inulin: Beneo Orafti Inulin
cfu count was performed by use of ISO 20391-1:2018.

### Example 1 (E1) - Comparative Examples 1-2 (CE1-CE3) - Cleaning product

An all-purpose cleaning product was defined, having one of the following compositions, as summarized in Table 1:

**Table 1 - compositions cleaning product**

| **Component** | CE1 | CE2 | CE3 | E1 |
|---|---|---|---|---|
| Water | 91,8 | 10 | 91,8 | 77,1 |
| Sodium Laureth Sulphate | 5,5 | 0 | 5,5 | 5,5 |
| Probiotic Mixture | 0 | 0 | 1 | 1 |
| Phenoxyethanol | 0,45 | 0 | 0,45 | 0,45 |
| Inulin | 0 | 0 | 0 | 0,3 |
| Galacto-oligosaccharide | 0 | 0 | 0 | 0,15 |
| Fructo-oligosaccharide | 0 | 0 | 0 | 0,15 |
| Cl 42051 | 0,05 | 0 | 0,05 | 0,05 |
| Fragrance | 0,3 | 0 | 0,3 | 0,3 |
| Ethanol | 0 | 90 | 0 | 0 |
| Isopropyl alcohol | 0 | 0 | 0 | 10 |
| Chloroxylenol | 0 | 0 | 0 | 5 |
| **Total weight (g)** | 98,1 | 100 | 99,1 | 100 |

wherein said components of said cleaning product are expressed in weight and wherein said Probiotic Mixture is an aqueous mixture of *Bacillus subtilis*, *Bacillus licheniformis*, *Bacillus megaterium* and *Bacillus amyloliquefaciens.* Each component of said probiotic mixture is present in a concentration of 2 x 10⁹ cfu per gram of the total composition, meaning that each component of said mixture has a number of spores which is 25% with reference to the total number of spores.

### Example 2 (E2) - Purification of a surface

All-purpose cleaning products according to the composition E1 (a synbiotic disinfectant), CE1 (forming a diluted detergent), CE2 (a concentrated disinfectant) and CE3 (a probiotic composition) were applied at a daily basis to a floor in a confined experimental space under controlled conditions for a total duration of 10 days.
Microbiological samples were taken from the surface of the floor in order to determine the presence of the hospital bacteria Staphylococcus aureus. Such samples were taken after 1, 6, 24, 48, 72, 120 and 240 hours. As of 24 hours, the samples were taken just before product application so as to measure the effect of the previous 24 hours of application.

The results in Figure 1 show the following:
- the application of the diluted detergent ("control" - cleaning product according to composition CE1) does not seem to affect the level of *S*. *aureus* CFU's.
- Applying the concentrated disinfectant ("disinfectant" - cleaning product according to composition CE2) results in a fast drop of *S*. *aureus* colony-forming units (CFU). However, this effect is only short-lived as the count for *S..aureus* rises back to the initial control level after a few hours.
- Applying the probiotic cleaning ("probiotic" - cleaning product according to composition CE3) results in a decline of *S*. *aureus* CFU's as of 24 hours in order to reach a minimum level (i.e. 430 cfu/m²) after 240 hours.
- Applying the synbiotic disinfectant ("synbiotic disinfectant" - cleaning product according to composition E4) leads to a direct and steep decline of *S*. *aureus* CFU's within the hour, wherein said decline is upheld for at least the next 10 days, resulting in a final level of 220 cfu/m² after 240 hours.

## Claims

1. A composition for the decontamination and inoculation of surfaces, said composition comprising an antimicrobial agent and a probiotic component, said probiotic component being a mixture of stabilized *Bacteria.*

2. The composition according to claim 1, wherein said probiotic component comprises at least one of *Bacillus* spores, Lactic Acid *Bacteria,* soil *Bacteria,* water *Bacteria,* human microbiome *Bacteria* or plant *Bacteria.*

3. The composition according to claim 1 or claim 2, wherein said probiotic component is a mixture of *Bacillus* spores.

4. The composition according to claim 3, wherein said mixture of *Bacillus* spores is present in a concentration of 1 x 10⁶ to 1 x 10¹⁰ cfu per gram composition.

5. The composition according to any one of previous claims, wherein said composition further comprises at least one prebiotic agent.

6. The composition according to claim 5, wherein said at least one prebiotic agent comprises at least one of a fructo-oligosaccharide, a galacto-oligosaccharide and/or inulin.

7. The composition according to claim 5 or claim 6, wherein said at least one prebiotic agent is present in a concentration of 100 mg to 100 g per kg composition.

8. The composition according to any one of previous claims, wherein the antimicrobial agent is selected from the group consisting of alcohols, aldehydes, oxidizing agents, peroxy acids, phenolics, quaternary ammonium compounds, chlorine compounds, iodine compounds, acid and bases, metals and terpenes, or mixtures thereof.

9. The composition according to any one of previous claims, wherein said antimicrobial agent is present in a concentration of 0,001 wt% to 90 wt% with reference to the total weight of the composition.

10. The composition according to any one claims 5-9, wherein said probiotic component and said at least one prebiotic agent are at least partly encapsulated in microcapsules.

11. The composition according to claim 10, wherein said microcapsule comprises a brittle shell and is furthermore filled with a non-aqueous solution.

12. The composition according to any one of previous claims, wherein said composition is formulated as a detergent, cleaning product, liquid, emulsion, cream, ointment, lotion, gel, oil, solution, aerosol spray, powder, or semi-solid formulation.

13. The composition according to any one of previous claims, wherein the surface is wood, paper or textile; wherein the surface is an abiotic material, such as stone, metal, plastic, glass, carbon, or ceramic; or wherein the surface is a composite material.

14. Method for treating surfaces with the composition for the simultaneous decontamination and inoculation of surfaces according to any one of claims 1-13, said method comprising the steps of cleaning, spraying, ultrasound fogging, painting, or water dosing said surfaces with said composition.

15. A cleaning product comprising the composition according to any one of claims 1-14.
